# EUROPEAN PATENT APPLICATION

(11) **EP 3 263 099 A1**
(43) Date of publication of application: **03.01.2018**
(21) Application number: 16176674.6
(22) Date of filing: 28.06.2016
(51) Int. Cl.: A61K 31/167, A61K 31/198, A61K 45/06, A61K 9/00, A61P 7/00, A61P 31/00, A61P 37/00, A61P 43/00

(54) **THYROID HORMONE DERIVATIVES FOR THE TREATMENT OF SEPSIS**

(71) Applicant: Universitätsklinikum Jena, 07743 Jena (DE)
(72) Inventor: BANHIAMAD, Aria, 07745 Jena (DE); KRAFT, Florian, 07745 Jena (DE)
(74) Representative: Schulz Junghans Patentanwälte PartGmbB

(57) **Abstract**

The present invention relates to a compound for use in a method for treating sepsis, severe sepsis or septic shock. The compound is selected from the group comprised of (2*S*)-2-amino-3-[4-(4-hydroxyphenoxy)-3,5-diiodo-phenyl]propanoic acid, 3-[3,5-dibromo-4-(4-hydroxy-3-isopropyl-phenoxy)anilino]-3-oxo-propanoic acid and (2*S*)-2-amino-3-[4-(4-hydroxy-3,5-diiodo-phenoxy)-3-iodo-phenyl]propanoic acid.

## Description

The present invention relates to derivatives and mimetics of the thyroid hormone for use in treating sepsis, severe sepsis or septic shock.

Sepsis is a severe disease caused by a complex systemic inflammation reaction after infection with microorganisms, caused by an overreaction of the immune system. In the course of sepsis life threating disorders of the vital function and failure of a single or multiple organs occur frequently. Sepsis has to be considered as a severe disease since approx. 30 to 50% of the affected patients die despite of maximal care. The 90-day morbidity of patients with severe sepsis and septic shock is 54%.

Additionally, sepsis is the third most frequent cause of death with approx. 60.000 cases of death each year after acute myocardial infarction and heart insufficiency in Germany, whereby roughly 30% of the expenses for intensive care are spent on treatment of sepsis (Deutsche-Sepsis Gesellschaft, Methodenreport; Brunkhorst, 2006). As a consequence, the supply with drugs to increase the survival rate of patients suffering sepsis would be highly desirable.

The influence of various hormones on the regulation of the immune system is well known. Of special note is the ability of thyroid hormone and thyroid stimulating hormone, which are not only known to modulate immune responses, but have been reported to be present in lower amounts in non-survivors of sepsis compared to survivors (Yildizdas et al., 2004; JPEM 17; 1435-1442). A possible beneficial effect of thyroid hormone treatment for sepsis survival is not known.

The thyroid hormone is known to positively affect a multitude of physiological parameters, but has also undesirable side effects such as increase of cardiac output and heart rate, therefore the thyroid hormone itself is rather unsuitable as a pharmaceutical. Interestingly, the undesired side effect of tachycardia seems to not be mediated by the thyronine receptors (Kahaly et al. 2005, Endocrine Rewievs 26(5), 704-728). However, various metabolites and derivatives of thyroid hormone with varying activity exist.

The thyroid gland produces predominantly the prohormone T4 (thyroxine) together with a small amount of the bioactive thyroid hormone T3 (triiodothyronine). Most T3 is produced by enzymatic outer ring deiodination (ORD) of T4 in peripheral tissues. Alternatively, inner ring deiodination (IRD) of T4 yields the metabolite rT3. T3 is further metabolized largely by IRD and rT3 largely by ORD, yielding in both cases the metabolite 3,5 T2 (diiodothyronine). Thus, ORD is regarded as an activating pathway and IRD as an inactivating pathway.

Recently efforts have been made to synthesize thyroid hormone mimetics that are more suitable as pharmaceuticals by eliminating their detrimental effect on physiological parameters such as heart rate while maintaining their beneficial effects on other parameters.

The problem underlying the present invention is to provide compounds suitable for the treatment of sepsis. This problem is solved by the subject-matter of the independent claims.

The surprising finding was made that treatment with the compounds of the invention results in improved survival rates after sepsis, wherein the aforementioned compounds are derivatives or mimetics of the thyroid hormone.

According to a first aspect of the invention, a compound for use in a method for treating sepsis, severe sepsis or septic shock is provided. The compound is selected from the group comprised of
- (2*S*)-2-amino-3-[4-(4-hydroxyphenoxy)-3,5-diiodo-phenyl]propanoic acid (3,5-T2, CAS No 1041-01-6): 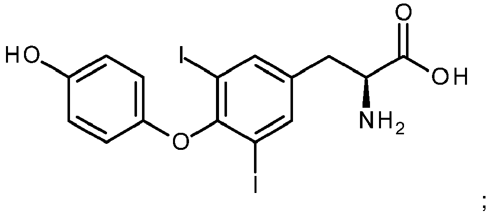
- 3-[3,5-dibromo-4-(4-hydroxy-3-isopropyl-phenoxy)anilino]-3-oxo-propanoic acid (KB2115, CAS No 355129-15-6): 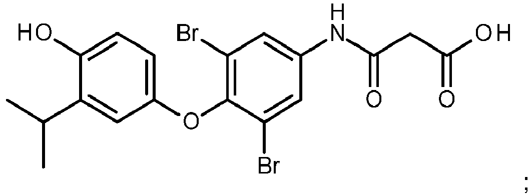 and
- (2*S*)-2-amino-3-[4-(4-hydroxy-3,5-diiodo-phenoxy)-3-iodo-phenyl]propanoic acid (rT3, CAS No 5817-39-0):

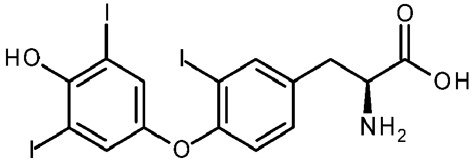

In certain embodiments, a combination of compounds is provided for use in a method for treating sepsis, severe sepsis or septic shock, wherein the combination of compounds comprises:
- (2*S*)-2-amino-3-[4-(4-hydroxyphenoxy)-3,5-diiodo-phenyl]propanoic acid and 3-[3,5-dibromo-4-(4-hydroxy-3-isopropyl-phenoxy)anilino]-3-oxo-propanoic acid, or
- (2*S*)-2-amino-3-[4-(4-hydroxyphenoxy)-3,5-diiodo-phenyl]propanoic acid and (2*S*)-2-amino-3-[4-(4-hydroxy-3,5-diiodo-phenoxy)-3-iodo-phenyl]propanoic acid, or
- 3-[3,5-dibromo-4-(4-hydroxy-3-isopropyl-phenoxy)anilino]-3-oxo-propanoic acid and (2*S*)-2-amino-3-[4-(4-hydroxy-3,5-diiodo-phenoxy)-3-iodo-phenyl]propanoic acid, or
- (2*S*)-2-amino-3-[4-(4-hydroxyphenoxy)-3,5-diiodo-phenyl]propanoic acid, 3-[3,5-dibromo-4-(4-hydroxy-3-isopropyl-phenoxy)anilino]-3-oxo-propanoic acid and (2*S*)-2-amino-3-[4-(4-hydroxy-3,5-diiodo-phenoxy)-3-iodo-phenyl]propanoic acid).

Advantageously, the compounds of the invention do not affect the cardiac output and/or the heart rate. Thus, the compounds of the invention are more beneficial and very suitable for the use as pharmaceuticals in general and for the proposed treatment in particular. Particularly, rT3 decreases the binding capacity of the beta adrenergic receptors at the heart, whereby T3 and T4 increase the aforementioned binding capacity.

In certain embodiments, the compound of the invention is administered according to the following dosage regimen:
a) administering an initial dose of the compound of the invention to a patient on the first day of treatment.

The term "first day of treatment" in the context of the present specification refers to 12 to 24 hours of treatment with the compound of the invention, wherein particularly the first day of treatment starts with the administration of the initial dose. The treatment may be preceded by a different treatment of sepsis, severe sepsis or septic shock without administering the compound of the invention.

In certain embodiments, the initial dose is administered immediately after or less than 30 min, 1 h or 2 hours after positive diagnosis of sepsis, severe sepsis or septic shock in the patient.

In certain embodiments, the initial dose is
- 1.5 µg to 4 mg per kg body weight of the patient of (2*S*)-2-amino-3-[4-(4-hydroxyphenoxy)-3,5-diiodo-phenyl]propanoic acid,
- 15 µg to 400 µg per kg body weight of the patient of (2*S*)-2-amino-3-[4-(4-hydroxy-3,5-diiodo-phenoxy)-3-iodo-phenyl]propanoic acid, and/or
- 0.3 µg to 400 µg per kg body weight of the patient of 3-[3,5-dibromo-4-(4-hydroxy-3-isopropyl-phenoxy)anilino]-3-oxo-propanoic acid.

In certain embodiments, the initial dose is
- 2 µg to 2 mg per kg body weight of the patient of (2*S*)-2-amino-3-[4-(4-hydroxyphenoxy)-3,5-diiodo-phenyl]propanoic acid, particularly 20 µg to 200 µg per kg body weight of the patient of (2S)-2-amino-3-[4-(4-hydroxyphenoxy)-3,5-diiodo-phenyl]propanoic acid,
- 20 µg to 200 µg per kg body weight of the patient of (2*S*)-2-amino-3-[4-(4-hydroxy-3,5-diiodo-phenoxy)-3-iodo-phenyl]propanoic acid, particularly 40 µg to 100 µg per kg body weight of the patient of (2*S*)-2-amino-3-[4-(4-hydroxy-3,5-diiodo-phenoxy)-3-iodo-phenyl]propanoic acid, and/or
- 1.5 µg to 200 µg per kg body weight of the patient of 3-[3,5-dibromo-4-(4-hydroxy-3-isopropyl-phenoxy)anilino]-3-oxo-propanoic acid, particularly 7.5 µg to 40 µg per kg body weight of the patient of 3-[3,5-dibromo-4-(4-hydroxy-3-isopropyl-phenoxy)anilino]-3-oxo-propanoic acid.

In certain embodiments, the dosage regimen further comprises:
b) administering an additional dose of the compound of the invention to the patient on the first day of treatment 8 to 14 hours after administering the initial dose.

In certain embodiments, the additional dose of the compound of the invention is administered 12 hours after administering the initial dose.

In certain embodiments, the additional dose is
- 1.5 µg to 4 mg per kg body weight of the patient of (2*S*)-2-amino-3-[4-(4-hydroxyphenoxy)-3,5-diiodo-phenyl]propanoic acid,
- 15 µg to 400 µg per kg body weight of the patient of (2*S*)-2-amino-3-[4-(4-hydroxy-3,5-diiodo-phenoxy)-3-iodo-phenyl]propanoic acid, and/or
- 0.3 µg to 400 µg per kg body weight of the patient of 3-[3,5-dibromo-4-(4-hydroxy-3-isopropyl-phenoxy)anilino]-3-oxo-propanoic acid.

In certain embodiments, the additional dose is
- 2 µg to 2 mg of per kg body weight of the patient (2*S*)-2-amino-3-[4-(4-hydroxyphenoxy)-3,5-diiodo-phenyl]propanoic acid, particularly 20 µg to 200 µg per kg body weight of the patient of (2*S*)-2-amino-3-[4-(4-hydroxyphenoxy)-3,5-diiodo-phenyl]propanoic acid,
- 20 µg to 200 µg per kg body weight of the patient of (2*S*)-2-amino-3-[4-(4-hydroxy-3,5-diiodo-phenoxy)-3-iodo-phenyl]propanoic acid, particularly 40 µg to 100 µg per kg body weight of the patient of (2*S*)-2-amino-3-[4-(4-hydroxy-3,5-diiodo-phenoxy)-3-iodophenyl]propanoic acid, and/or
- 1.5 µg to 200 µg per kg body weight of the patient of 3-[3,5-dibromo-4-(4-hydroxy-3-isopropyl-phenoxy)anilino]-3-oxo-propanoic acid, particularly 7.5 µg to 40 µg per kg body weight of the patient of 3-[3,5-dibromo-4-(4-hydroxy-3-isopropyl-phenoxy)anilino]-3-oxo-propanoic acid.

In certain embodiments, the dosage regimen further comprises
c) administering a first additional dose of the compound of the invention to the patient on the second day of treatment.

The term "second day of treatment" in the context of the present specification refers to 24 hours of treatments that follows the first day of treatment.

In certain embodiments, the first additional dose of step c) is administered 8 to 14 hours after administering the additional dose of step b). In certain embodiments, the first additional dose of step c) is administered 12 hours after administering the additional dose of step b).

In certain embodiments, the dosage regimen further comprises:
d) administering a second additional dose of the compound of the invention to the patient on the second day of treatment 8 to 14 hours after administering the first additional dose of step c).

In certain embodiments, the second additional dose of step d) is administered 12 hours after administering the first additional dose of step c).

In certain embodiments, the dosage regimen further comprises:
e) administering a first additional dose of the compound of the invention to the patient on the third day of treatment.

The term "third day of treatment" in the context of the present specification refers to 24 hours of treatments that follow the second day of treatment.

In certain embodiments, the first additional dose of step e) is administered 8 to 14 hours after administering the second additional dose of step d). In certain embodiments, the first additional dose of step e) is administered 12 hours after administering the additional dose of step d).

In certain embodiments, the dosage regimen further comprises:
f) administering a second additional dose of the compound of the invention to the patient on the third day of treatment 8 to 14 hours after administering the first additional dose of step e).

In certain embodiments, the second additional dose of step f) is administered 12 hours after administering the first additional dose of step e).

In certain embodiments, the dosage regimen further comprises:
g) administering a first additional dose of the compound of the invention to the patient on each subsequent day of treatment following the third day of treatment, and optionally
h) administering a second additional dose of the compound of the invention to the patient on each subsequent day of treatment following the third day of treatment 8 to 14 hours after administering the first additional dose of step g).

In certain embodiments, the first additional dose of step g) is administered 8 to 14 hours after administering the second additional dose of step f) or step h) of the preceding day of treatment. In certain embodiments, the first additional dose of step g) is administered 12 hours after administering the second additional dose of steps f) or step h) of the preceding day of treatment.

In certain embodiments, the second additional dose of step h) is administered 12 hours after administering the first additional dose of step g).

In certain embodiments, the compound of the invention is administered on the fourth day, the firth day, the sixth day and/or the seventh day of treatment according to the dosage regimen described above.

In certain embodiments, the first additional dose of step c) and/or step e) and/or step g) is
- 1.5 µg to 4 mg per kg body weight of the patient of (2*S*)-2-amino-3-[4-(4-hydroxyphenoxy)-3,5-diiodo-phenyl]propanoic acid,
- 15 µg to 400 µg per kg body weight of the patient of (2*S*)-2-amino-3-[4-(4-hydroxy-3,5-diiodo-phenoxy)-3-iodo-phenyl]propanoic acid, and/or
- 0.3 µg to 400 µg per kg body weight of the patient of 3-[3,5-dibromo-4-(4-hydroxy-3-isopropyl-phenoxy)anilino]-3-oxo-propanoic acid.

In certain embodiments, the first additional dose of step c) and/or step e) and/ or step g) is
- 2 µg to 2 mg per kg body weight of the patient of (2*S*)-2-amino-3-[4-(4-hydroxyphenoxy)-3,5-diiodo-phenyl]propanoic acid, particularly 20 µg to 200 µg per kg body weight of the patient of (2*S*)-2-amino-3-[4-(4-hydroxyphenoxy)-3,5-diiodo-phenyl]propanoic acid,
- 20 µg to 200 µg kg body weight of the patient of (2*S*)-2-amino-3-[4-(4-hydroxy-3,5-diiodo-phenoxy)-3-iodo-phenyl]propanoic acid, particularly 40 µg to 100 µg per kg body weight of the patient of (2*S*)-2-amino-3-[4-(4-hydroxy-3,5-diiodo-phenoxy)-3-iodophenyl]propanoic acid, and/or
- 1.5 µg to 200 µg per kg body weight of the patient of 3-[3,5-dibromo-4-(4-hydroxy-3-isopropyl-phenoxy)anilino]-3-oxo-propanoic acid, particularly 7.5 µg to 40 µg per kg body weight of the patient of 3-[3,5-dibromo-4-(4-hydroxy-3-isopropyl-phenoxy)anilino]-3-oxo-propanoic acid.

In certain embodiments, the second additional dose of step d) and/or step f) and/or step h) is
- 1.5 µg to 4 mg per kg body weight of the patient of (2*S*)-2-amino-3-[4-(4-hydroxyphenoxy)-3,5-diiodo-phenyl]propanoic acid,
- 15 µg to 400 µg per kg body weight of the patient of (2*S*)-2-amino-3-[4-(4-hydroxy-3,5-diiodo-phenoxy)-3-iodo-phenyl]propanoic acid, and/or
- 0.3 µg to 400 µg per kg body weight of the patient of 3-[3,5-dibromo-4-(4-hydroxy-3-isopropyl-phenoxy)anilino]-3-oxo-propanoic acid.

In certain embodiments, the second additional dose of step d) and/or step f) and/or step h) is
- 2 µg to 2 mg per kg body weight of the patient of (2*S*)-2-amino-3-[4-(4-hydroxyphenoxy)-3,5-diiodo-phenyl]propanoic acid, particularly 20 µg to 200 µg kg body weight of the patient of (2*S*)-2-amino-3-[4-(4-hydroxyphenoxy)-3,5-diiodo-phenyl]propanoic acid,
- 20 µg to 200 µg per kg body weight of the patient of (2*S*)-2-amino-3-[4-(4-hydroxy-3,5-diiodo-phenoxy)-3-iodo-phenyl]propanoic acid, particularly 40 µg to 100 µg per kg body weight of the patient of (2*S*)-2-amino-3-[4-(4-hydroxy-3,5-diiodo-phenoxy)-3-iodophenyl]propanoic acid, and/or
- 1.5 µg to 200 µg kg body weight of the patient of 3-[3,5-dibromo-4-(4-hydroxy-3-isopropyl-phenoxy)anilino]-3-oxo-propanoic acid, particularly 7.5 µg to 40 µg kg body weight of the patient of 3-[3,5-dibromo-4-(4-hydroxy-3-isopropyl-phenoxy)anilino]-3-oxo-propanoic acid.

In certain embodiments, the compound of the invention is orally administered. In certain embodiments, the compound of the invention is administered intraperitoneally. In certain embodiments, the compound of the invention is administered subcutaneously.

According to another aspect of the invention, a pharmaceutical composition for use in a method for treating sepsis, severe sepsis or septic shock is provided. The pharmaceutical composition comprises (2*S*)-2-amino-3-[4-(4-hydroxyphenoxy)-3,5-diiodo-phenyl]propanoic acid, 3-[3,5-dibromo-4-(4-hydroxy-3-isopropyl-phenoxy)anilino]-3-oxo-propanoic acid and/or (2S)-2-amino-3-[4-(4-hydroxy-3,5-diiodo-phenoxy)-3-iodo-phenyl]propanoic acid.

Similarly, a dosage form for the treatment of sepsis, severe sepsis or septic shock is provided, comprising (2*S*)-2-amino-3-[4-(4-hydroxyphenoxy)-3,5-diiodo-phenyl]propanoic acid, 3-[3,5-dibromo-4-(4-hydroxy-3-isopropyl-phenoxy)anilino]-3-oxo-propanoic acid and/or (2*S*)-2-amino-3-[4-(4-hydroxy-3,5-diiodo-phenoxy)-3-iodo-phenyl]propanoic acid. Dosage forms may be for enteral administration, such as nasal, buccal, rectal, transdermal or oral administration, or as an inhalation form or suppository. Alternatively, parenteral administration may be used, such as subcutaneous, intravenous, intrahepatic or intramuscular injection forms. Optionally, a pharmaceutically acceptable carrier and/or excipient may be present.

In certain embodiments, (2*S*)-2-amino-3-[4-(4-hydroxyphenoxy)-3,5-diiodo-phenyl]propanoic acid, 3-[3,5-dibromo-4-(4-hydroxy-3-isopropyl-phenoxy)anilino]-3-oxo-propanoic acid and/or (2S)-2-amino-3-[4-(4-hydroxy-3,5-diiodo-phenoxy)-3-iodo-phenyl]propanoic acid are applied as an oral formulation, nasal inhalant, injection form, suppository or topical formulation, wherein an oral formulation or an injection form is preferred.

In certain embodiments, the dosage form comprises
- 1.5 µg to 4 mg per kg body weight of a patient of (2*S*)-2-amino-3-[4-(4-hydroxyphenoxy)-3,5-diiodo-phenyl]propanoic acid,
- 15 µg to 400 µg per kg body weight of a patient of (2S)-2-amino-3-[4-(4-hydroxy-3,5-diiodo-phenoxy)-3-iodo-phenyl]propanoic acid, and/or
- 0.3 µg to 400 µg per kg body weight of a patient of 3-[3,5-dibromo-4-(4-hydroxy-3-isopropyl-phenoxy)anilino]-3-oxo-propanoic acid.

In certain embodiments, the dosage form comprises:
- 2 µg to 2 mg per kg body weight of a patient of (2*S*)-2-amino-3-[4-(4-hydroxyphenoxy)-3,5-diiodo-phenyl]propanoic acid, particularly 20 µg to 200 µg per kg body weight of a patient of (2S)-2-amino-3-[4-(4-hydroxyphenoxy)-3,5-diiodo-phenyl]propanoic acid,
- 20 µg to 200 µg per kg body weight of a patient of (2*S*)-2-amino-3-[4-(4-hydroxy-3,5-diiodo-phenoxy)-3-iodo-phenyl]propanoic acid, particularly 40 µg to 100 µg per kg body weight of a patient of (2*S*)-2-amino-3-[4-(4-hydroxy-3,5-diiodo-phenoxy)-3-iodo-phenyl]propanoic acid, and/or
- 1.5 µg to 200 µg per kg body weight of a patient of 3-[3,5-dibromo-4-(4-hydroxy-3-isopropyl-phenoxy)anilino]-3-oxo-propanoic acid, particularly 7.5 µg to 40 µg per kg body weight of a patient of 3-[3,5-dibromo-4-(4-hydroxy-3-isopropyl-phenoxy)anilino]-3-oxo-propanoic acid.

According to another aspect of the invention, a method for treating sepsis, severe sepsis or septic shock is provided. The method comprises the administration of (2*S*)-2-amino-3-[4-(4-hydroxyphenoxy)-3,5-diiodo-phenyl]propanoic acid, 3-[3,5-dibromo-4-(4-hydroxy-3-isopropyl-phenoxy)anilino]-3-oxo-propanoic acid and/or (2S)-2-amino-3-[4-(4-hydroxy-3,5-diiodo-phenoxy)-3-iodo-phenyl]propanoic acid to a patient in need thereof.

In certain embodiments, (2*S*)-2-amino-3-[4-(4-hydroxyphenoxy)-3,5-diiodo-phenyl]propanoic acid, 3-[3,5-dibromo-4-(4-hydroxy-3-isopropyl-phenoxy)anilino]-3-oxo-propanoic acid and/or (2S)-2-amino-3-[4-(4-hydroxy-3,5-diiodo-phenoxy)-3-iodo-phenyl]propanoic acid are administered according to the above described dosage regimen.

According to another aspect of the invention, a method for manufacturing a medicament for treating sepsis, severe sepsis or septic shock is provided. The method comprises the use of (2*S*)-2-amino-3-[4-(4-hydroxyphenoxy)-3,5-diiodo-phenyl]propanoic acid, 3-[3,5-dibromo-4-(4-hydroxy-3-isopropyl-phenoxy)anilino]-3-oxo-propanoic acid and/or (2*S*)-2-amino-3-[4-(4-hydroxy-3,5-diiodo-phenoxy)-3-iodo-phenyl]propanoic acid for the manufacturing of a medicament for treating sepsis. Medicaments according to the invention are manufactured by methods known in the art, especially by conventional mixing, coating, granulating, dissolving or lyophilizing.

The invention is further illustrated by the following examples and figures, from which further embodiments and advantages can be drawn. These examples are meant to illustrate the invention but not to limit its scope.

### Description of the figures

- Fig. 1: shows the activation of both thyroid receptors by 3,5-diiodothyronine (3,5-T2), wherein the luciferase (RLU) activities are plotted against the indicated concentrations of 3,5-T2; Error bars represent deviations from the mean.
- Fig. 2: shows the activation of both thyroid receptors by T3, wherein the luciferase (RLU) activities are plotted against the indicated concentrations of T3; Error bars represent deviations from the mean.
- Fig. 3: shows the effect of 3,5-diiodothyonine (3,5-T2) on the survival of septic mice in a Kaplan-Meier survival curve, wherein the percentage survival of control-treated mice (light grey) and mice treated with 3,5-T2 (black) is plotted against the time after sepsis induction.
- Fig. 4: shows the activation of both thyroid receptors by KB2115, wherein the luciferase (RLU) activities are plotted against the indicated concentrations of KB2115; Error bars represent deviations from the mean.
- Fig. 5: shows the effect of KB2115 on the survival of septic mice in a Kaplan-Meier survival curve, wherein the percentage survival of control-treated mice (light grey) and mice treated with KB2115 (black) is plotted against the time after sepsis induction.
- Fig. 6: shows the activation of both thyroid receptors by rT3, wherein the luciferase (RLU) activities are plotted against the indicated concentrations of rT3; Error bars represent deviations from the mean.
- Fig. 7: shows the effect of rT3 on the survival of septic mice in a Kaplan-Meier survival curve, wherein the percentage survival of untreated mice (light grey) and mice treated with rT3 (black) is plotted against the time after sepsis induction.
- Fig. 8: shows Box-plots of the T3 level in septic mice treated with the indicated compounds.

### Examples

### Methods and materials:

For testing the efficacy of the compounds of the invention for treating sepsis, the PCI mouse model (peritoneal contamination and injection) was used, wherein human feces are injected into the abdomen of the mice inducing sepsis with human sepsis pathogens. This renders the animal model superior for studying sepsis and severe sepsis and particularly for testing compounds for treating human sepsis.

At least 10 weeks old, male mature wild type mice (C57BL/6 J) were weighed, and a suspension of human feces (1.25 µl per g body weight) were injected i.p. Subsequently, the mice received a subcutaneous injection of the respective compound of the invention in the back of the neck and were transferred back into their cages. The mice received additional injections after the indicated periods. Mice receiving solvent injection (PBS buffer) were used as control.

### Example 1: Effect of 3,5-diiodothyronine (3,5-T2)

### In vitro experiments

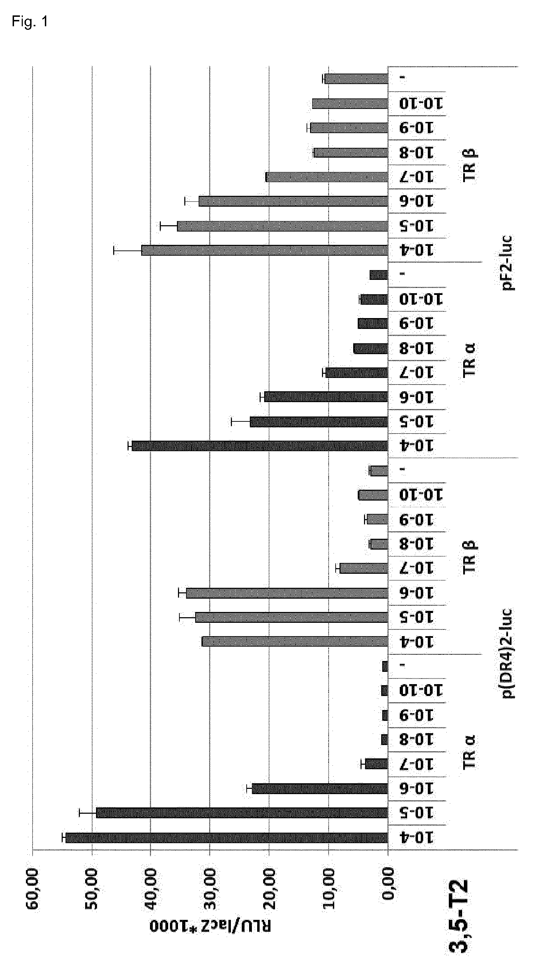

The effect of 3,5-T2 on the thyroid hormone (TR) receptors was demonstrated in CV1 cells lacking endogenous TRs. CV1 cells were co-transfected with (0.2µg) expression vectors for either TR alpha or TR beta together with a reporter plasmid (1 µg). The reporter plasmid contained a luciferase gene either under the control of the perfect palindromic DR4 sequence or the natural F2 sequence. The plasmid pCMV LacZ was used as an internal control for the transfection efficiency. The transfected cells were treated with the indicated concentrations of 3,5-T2 ranging from 10⁻¹⁰ to 10⁻⁴ mol*l⁻¹ (Fig. 1). After 72 hours cells were lysed and both the luciferase (RLU; relative light units) and beta-galactosidase activities (LacZ) were measured. The obtained RLU values were normalized to the values of LacZ to correct for possible differences in transfection efficiency. Increased thyroid hormone receptor activity results in an increased expression of the luciferase reporter. Luciferase activity increased with increasing concentrations of 3,5-T2 both in cells transfected with TR alpha or TR beta (Fig. 1) indicating an increased activation of both thyroid receptors by 3,5-T2. This effect was observed in cells transfected with the palindromic DR4 sequence and the natural F2 sequence.

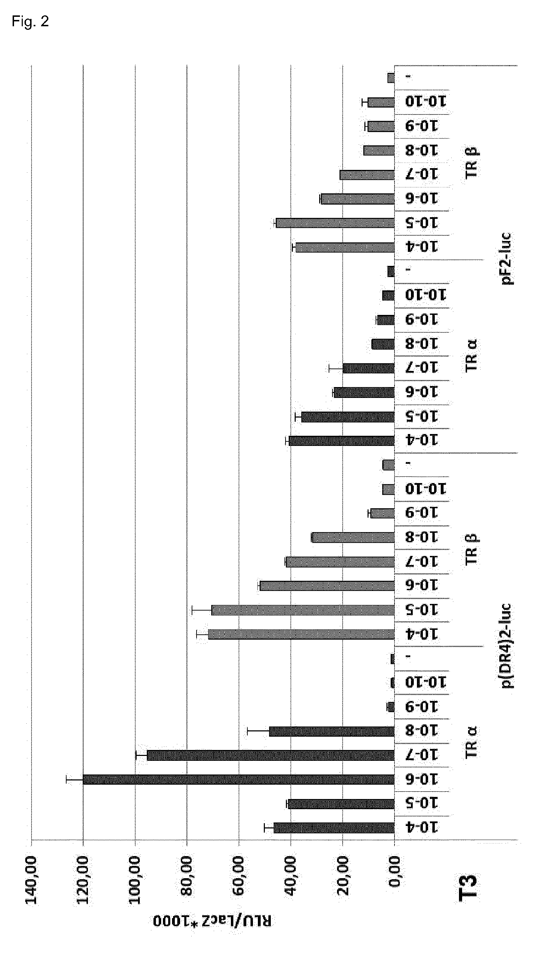

As a positive control, the experiments with the transfected CV1 cells were repeated with triidothyronine (T3). For that, the transfected cells were treated with the indicated concentrations of triiodothyronine ranging from 10⁻¹⁰ to 10⁻⁴ mol*l⁻¹ (Fig. 2). After 72 hours the cells were lysed and both the luciferase (RLU) and beta-galactosidase activities were measured. The obtained RLU values were normalized to the values of LacZ.

### In vivo experiments

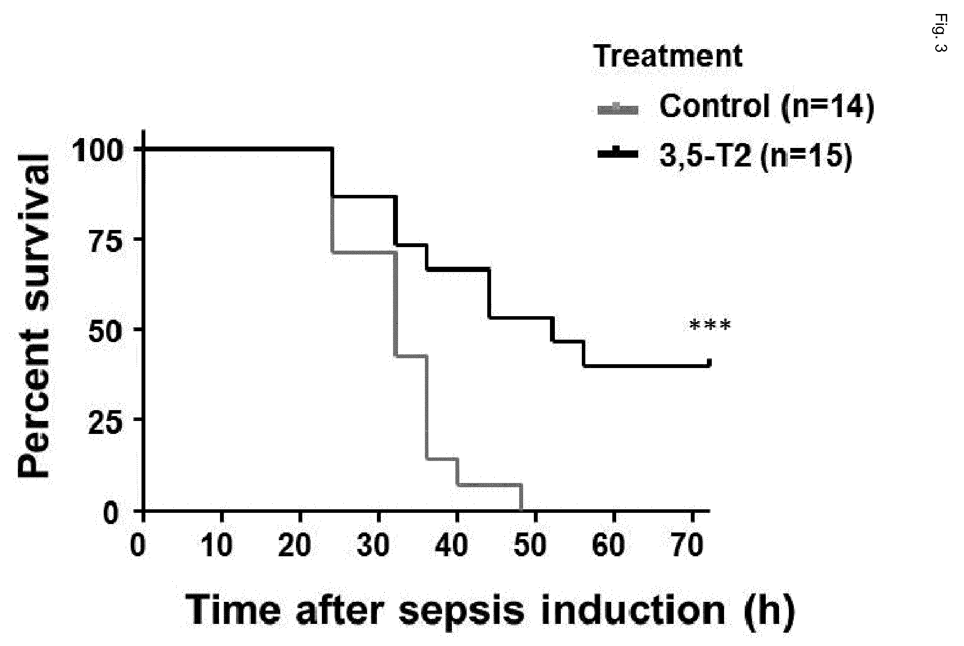

Sepsis was induced using the PCI mouse model system as described above. 3,5-diiodothyronine (3,5-T2, 4 mg/kg body weight) was injected after 0, 12, 24, 36, 48 and 60 hours. The survival rate of the mice is shown in the Kaplan-Meier curve depicted in Fig 3. The solvent PBS was used as the control treatment. Statistical significance of the treatment was calculated by the Mantel-Cox test. Administration of 3,5-T2 significantly improved the survival of mice suffering severe sepsis, wherein 40% of the mice survived after 72 h in contrast to no survivors in the control group.

### Example 2: Effect of KB2115

### In vitro experiments

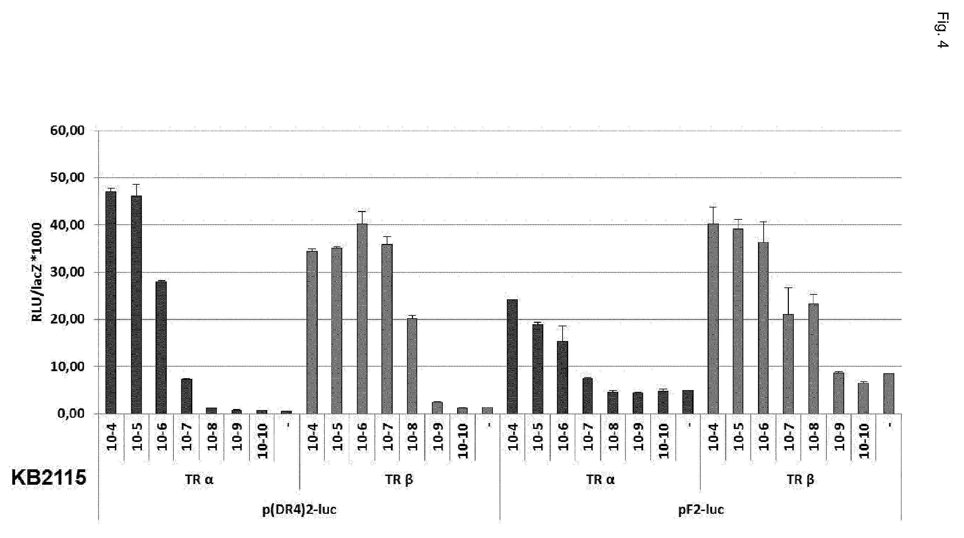

The effect of KB2115 on thyroid hormone receptors was demonstrated in transfected CV1 cells as described in example 1. Briefly, the transfected cells were treated with the indicated concentrations of KB2115 ranging from 10⁻¹⁰ to 10⁻⁴ mol*l⁻¹ (Fig. 4). After 72 hours the cells were lysed and both the luciferase (RLU) and beta-galactosidase activities (LacZ) were measured. The obtained RLU values were normalized to the values of LacZ. Increased thyroid hormone receptor activity results in an increased expression of the luciferase reporter. As a result, the luciferase activity increased with increasing concentrations of KB2115 (Fig. 4) indicating an increased activation of both thyroid hormone receptors by KB2115. This effect was observed in cells transfected with the palindromic DR4 sequence and the natural F2 sequence.

### In vivo experiments

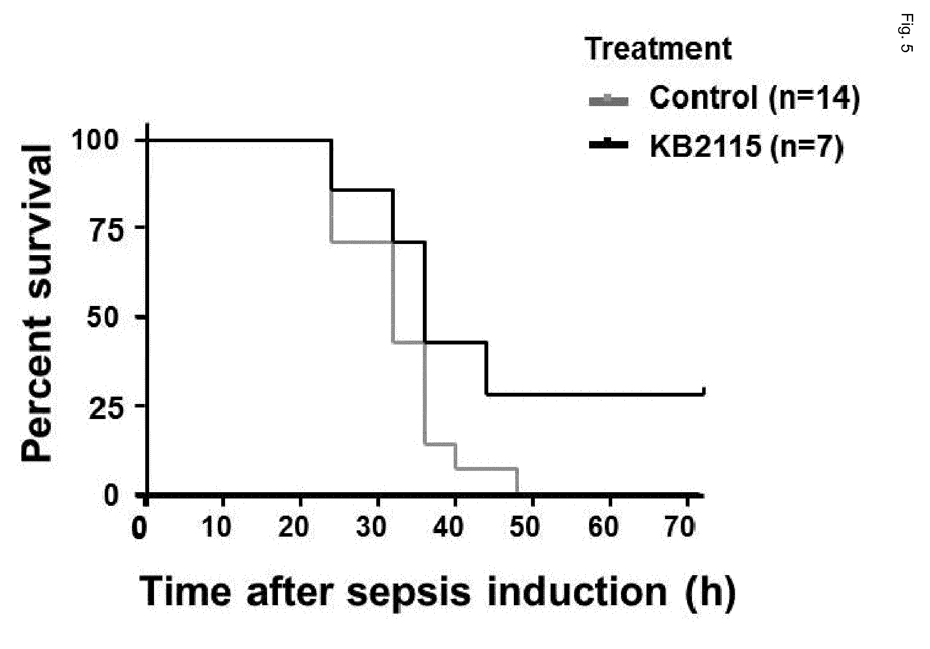

Sepsis was induced using the PCI mouse model system as described above. KB2115 (0.4 mg/kg body weight) was injected after 0, 12, 24, 36, 48 and 60 hours. The survival rate of the mice is shown in the Kaplan-Meier curve depicted in Fig 5. The solvent PBS was used as the control treatment. Statistical significance of the treatment was tested by the Mantel-Cox test yielding a p-value of 0.638. By administration of KB2115 the survival of mice suffering severe sepsis could be improved. Survival could be observed in 25% of the mice treated with KB2115 in contrast to no survivors in the control group.

### Example 3: Effect of rT3

### In vitro experiments

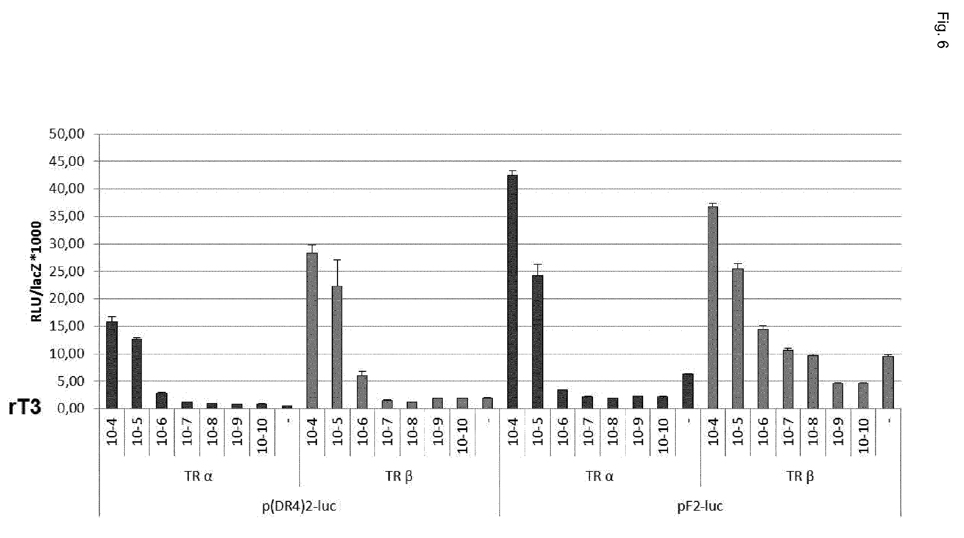

The effect of rT3 on the thyroid hormone receptors was demonstrated in transfected CV1 cells according to example 1. The transfected cells were treated with the indicated concentrations of rT3 ranging from 10⁻¹⁰ to 10⁻⁴ mol*l⁻¹ (Fig. 6). After 72 hours the cells were lysed and both the luciferase (RLU) and beta-galactosidase activities (LacZ) were measured. The obtained RLU values were normalized to the values of LacZ. Increased thyroid hormone receptor activity results in an increased expression of the luciferase reporter. As a result, the luciferase activity increased with increasing concentrations of rT3 (Fig. 6) indicating an increased activation of both thyroid hormone receptors by rT3. This effect was observed in cells transfected with the palindromic DR4 sequence and the natural F2 sequence.

### In vivo experiments

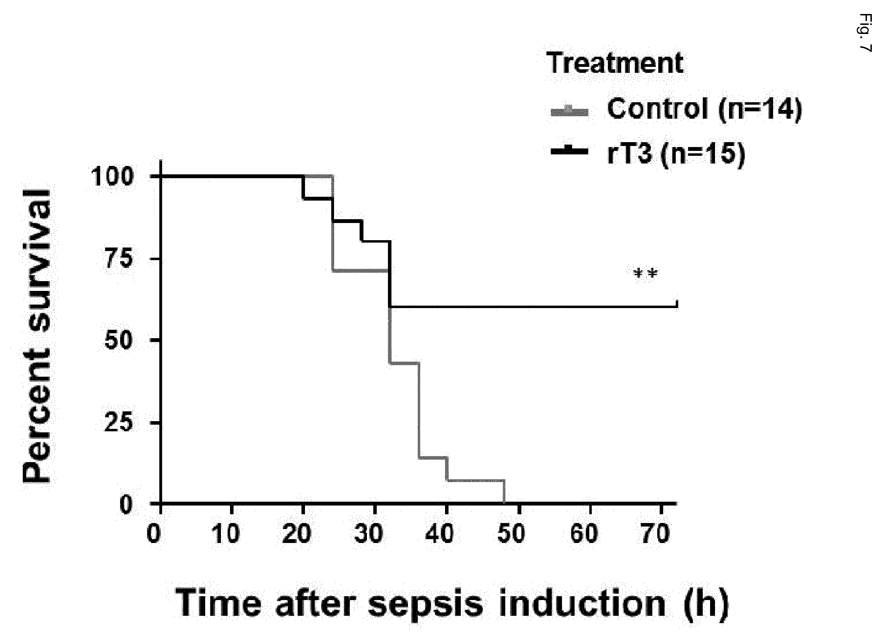

Sepsis was induced using the PCI mouse model system as described above. rT3 (0.4 mg/kg body weight) was injected after 0, 12, 24, 36, 48 and 60 hours. The survival rate of the mice is shown in the Kaplan-Meier curve depicted in Fig 7. The solvent PBS was used as the control treatment. Statistical significance of the treatment was calculated by the Mantel-Cox test. By administration of rT3 the survival of mice suffering severe sepsis could significantly be improved, wherein a survival could be observed in >50% of the mice in contrast to no survivors in the control group.

### Example 4: Effect of administration of the compounds of the invention on the T3 levels

In further experiments, the question was examined, whether the treatment of septic mice with the compounds of the invention up-regulates the endogenous level of T3, and by that may increase the survival after sepsis.

Accordingly, the T3 levels of septic mice were determined after treatment with the compounds of the invention. For that, sera of the septic mice after treatment were collected, and the T3 levels in the sera were determined.

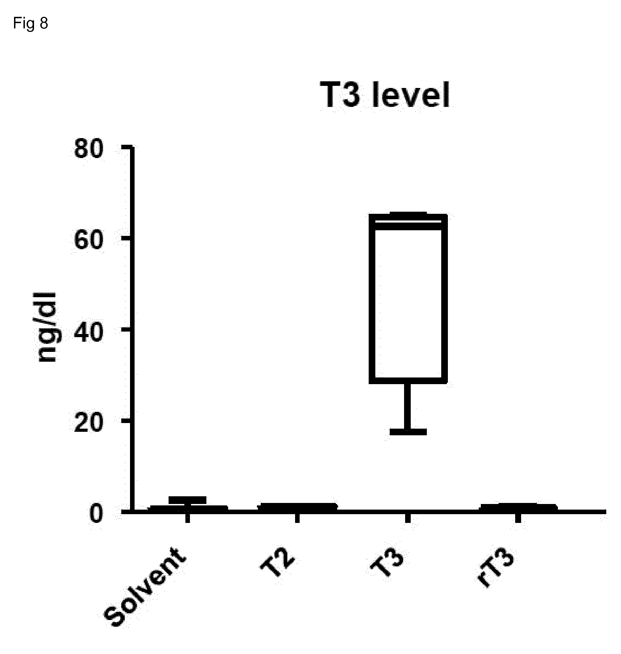

As depicted in Fig. 8, treatment of the mice with T3 expectedly resulted in an increase of the T3 level in the serum of the respective mice. However, treatment with T2 or rT3 did not result in an increase of the T3 level in the serum of the respective mice. Without wishing to be bound by theory, the inventors assume that the increased survival after sepsis mediated by the compounds of the invention is independent of the endogenous T3 level in the treated subject.

## Claims

1. A compound for use in a method for treating sepsis, severe sepsis or septic shock, **characterized in that** said compound is selected from the group comprised of
- (2S)-2-amino-3-[4-(4-hydroxyphenoxy)-3,5-diiodo-phenyl]propanoic acid,
- 3-[3,5-dibromo-4-(4-hydroxy-3-isopropyl-phenoxy)anilino]-3-oxo-propanoic acid, and
- (2*S*)-2-amino-3-[4-(4-hydroxy-3,5-diiodo-phenoxy)-3-iodo-phenyl]propanoic acid.

2. The compound for use in a method for treating sepsis, severe sepsis or septic shock, according to claim 1, comprising the dosage regimen:
a) administering an initial dose of said compound to a patient on the first day of treatment.

3. The compound for use in a method for treating sepsis, severe sepsis or septic shock according to claim 2, wherein said initial dose is
- 1.5 µg to 4 mg per kg body weight of said patient of (2*S*)-2-amino-3-[4-(4-hydroxyphenoxy)-3,5-diiodo-phenyl]propanoic acid,
- 15 µg to 400 µg per kg body weight of said patient of (2*S*)-2-amino-3-[4-(4-hydroxy-3,5-diiodo-phenoxy)-3-iodo-phenyl]propanoic acid, and/or
- 0.3 µg to 400 µg per kg body weight of said patient of 3-[3,5-dibromo-4-(4-hydroxy-3-isopropyl-phenoxy)anilino]-3-oxo-propanoic acid.

4. The compound for use in a method for treating sepsis, severe sepsis or septic shock according to claim 2 or 3, **characterized in that** said dosage regimen further comprises:
b) administering an additional dose of said compound to said patient on the first day of treatment 8 to 14 hours after administering said initial dose.

5. The compound for use in a method for treating sepsis, severe sepsis or septic shock according to claim 4, wherein said additional dose is
- 1.5 µg to 4 mg per kg body weight of said patient of (2*S*)-2-amino-3-[4-(4-hydroxyphenoxy)-3,5-diiodo-phenyl]propanoic acid,
- 15 µg to 400 µg per kg body weight of said patient of (2*S*)-2-amino-3-[4-(4-hydroxy-3,5-diiodo-phenoxy)-3-iodo-phenyl]propanoic acid, and/or
- 0.3 µg to 400 µg per kg body weight of said patient of 3-[3,5-dibromo-4-(4-hydroxy-3-isopropyl-phenoxy)anilino]-3-oxo-propanoic acid.

6. The compound for use in a method for treating sepsis, severe sepsis or septic shock according to any one of claims 2 to 5, wherein said dosage regimen further comprises:
c) administering a first additional dose of said compound to said patient on the second day of treatment, and optionally
d) administering a second additional dose of said compound to said patient on the second day of treatment 8 to 14 hours after administering said first additional dose of step c).

7. The compound for use in a method for treating sepsis, severe sepsis or septic shock according to claim 6, wherein said dosage regiment further comprises:
e) administering a first additional dose of said compound to said patient on the third day of treatment, and optionally
f) administering a second additional dose of said compound to said patient on the third day of treatment 8 to 14 hours after administering said first additional dose of step e).

8. The compound for use in a method for treating sepsis, severe sepsis or septic shock according to claim 6 or 7, wherein said first additional dose and/or said second additional dose is
- 1.5 µg to 4 mg per kg body weight of said patient of (2*S*)-2-amino-3-[4-(4-hydroxyphenoxy)-3,5-diiodo-phenyl]propanoic acid,
- 15 µg to 400 µg per kg body weight of said patient of (2*S*)-2-amino-3-[4-(4-hydroxy-3,5-diiodo-phenoxy)-3-iodo-phenyl]propanoic acid, and/or
- 0.3 µg to 400 µg per kg body weight of said patient of 3-[3,5-dibromo-4-(4-hydroxy-3-isopropyl-phenoxy)anilino]-3-oxo-propanoic acid.

9. A pharmaceutical composition for use in a method for treating sepsis, severe sepsis or septic shock, comprising (2*S*)-2-amino-3-[4-(4-hydroxyphenoxy)-3,5-diiodo-phenyl]propanoic acid, 3-[3,5-dibromo-4-(4-hydroxy-3-isopropyl-phenoxy)anilino]-3-oxo-propanoic acid and/or (2*S*)-2-amino-3-[4-(4-hydroxy-3,5-diiodo-phenoxy)-3-iodophenyl]propanoic acid.

10. A dosage form for treating sepsis, severe sepsis or septic shock, comprising (2*S*)-2-amino-3-[4-(4-hydroxyphenoxy)-3,5-diiodo-phenyl]propanoic acid, 3-[3,5-dibromo-4-(4-hydroxy-3-isopropyl-phenoxy)anilino]-3-oxo-propanoic acid and/or (2S)-2-amino-3-[4-(4-hydroxy-3,5-diiodo-phenoxy)-3-iodo-phenyl]propanoic acid.

11. The dosage form for treating sepsis, severe sepsis or septic shock according to claim 10, comprising
- 1.5 µg to 4 mg per kg body weight of a patient of (2*S*)-2-amino-3-[4-(4-hydroxyphenoxy)-3,5-diiodo-phenyl]propanoic acid,
- 15 µg to 400 µg per kg body weight of a patient of (2*S*)-2-amino-3-[4-(4-hydroxy-3,5-diiodo-phenoxy)-3-iodo-phenyl]propanoic acid, and/or
- 0.3 µg to 400 µg per kg body weight of a patient of 3-[3,5-dibromo-4-(4-hydroxy-3-isopropyl-phenoxy)anilino]-3-oxo-propanoic acid.

12. The dosage form for treating sepsis, severe sepsis or septic shock according to claim 10 or 11, wherein (2S)-2-amino-3-[4-(4-hydroxyphenoxy)-3,5-diiodo-phenyl]propanoic acid, 3-[3,5-dibromo-4-(4-hydroxy-3-isopropyl-phenoxy)anilino]-3-oxo-propanoic acid and/or (2S)-2-amino-3-[4-(4-hydroxy-3,5-diiodo-phenoxy)-3-iodo-phenyl]propanoic acid are applied as an oral formulation, nasal inhalant, injection form, suppository or topical formulation.
